# EUROPÄISCHE PATENTSCHRIFT

(11) **EP 3 389 550 B1**
(45) Veröffentlichungstag und Bekanntmachung des Hinweises auf die Patenterteilung: **10.03.2021**
(21) Anmeldenummer: 16822428.5
(22) Anmeldetag: 15.12.2016
(51) Int. Cl.: A61C 8/00

(54) **KERAMIKIMPLANTAT**
CERAMIC IMPLANT
IMPLANT EN CÉRAMIQUE

(30) Priorität: 17.12.2015 CH 18482015
(43) Veröffentlichungstag der Anmeldung: 24.10.2018
(73) Patentinhaber: Dentalpoint AG, 8048 Zürich (CH)
(72) Erfinder: BOLLETER, Philip, 8952 Schlieren (CH); WETTSTEIN, Pascal, 5726 Unterkulm (CH); WAHRHANEK, Maximilian, 8001 Zürich (CH)
(74) Vertreter: Rentsch Partner AG
(86) Internationale Anmeldenummer: PCT/EP2016/081317
(87) Internationale Veröffentlichungsnummer: WO 2017/103026

(56) Entgegenhaltungen:
- WO-A2-2008/022635
- US-A1- 2014 162 210

## Beschreibung

### TECHNISCHES GEBIET

Die Erfindung betrifft ein mindestens zweiteiliges Zahnersatzsystem aus Zirkondioxid mit einem ersten und einem zweiten zahnprothetischen Versorgungsteil, wobei die Eckradien dieser Versorgungsteile sehr kleine Eckradien aufweisen. Des Weiteren betrifft die Erfindung ein Verfahren zur Herstellung dieser Versorgungsteile.

### STAND DER TECHNIK

Aus dem Stand der Technik sind drei Bearbeitungstechniken für Zirkondioxid im Bereich Herstellung von Zahnersatzsystemen bekannt. Um das Implantat und die dazugehörigen Teile wie Abutments, Schrauben und Stifte in die geometrisch gewünschte Form zu bringen, erfolgt eine Hartbearbeitung des bereits gesinterten Grundkörpers durch Diamantschleifen.

Eine zweite Methode um den Zirkondioxidkörper in Form zu bringen, ist seine Bearbeitung als Grünkörper vor der Sinterung, was aufgrund der geringeren Härte des Materials einfacher ist. Es konnte jedoch festgestellt werden, dass es bei der nachfolgenden Sinterung zu einem Verzug des Zirkondioxids kommt und die geforderten Fertigungstoleranzen nicht mehr eingehalten werden konnten. Dies bedingt eine Nachbearbeitung des gesinterten Bauteils um die geforderten engen Fertigungstoleranzen realisieren zu können.

Vergleichbares gilt beim dritten, nach dem Stand der Technik, bekannten Verfahren einer Spritzgusstechnik, bei welcher nach dem Aushärten ein weiterer Bearbeitungsschritt, beispielsweise durch Schleifen erforderlich ist, um die notwendige Präzision in der Fertigung überhaupt erreichen zu können.

Die von der Anmelderin bisher realisierten Zahnimplantate und Abutments sind aus Zirkondioxid (hochfest, heiss-isostatisch gepresst und mit Yttrium dotiert) und weisen durch eine Hartbearbeitung des bereits gesinterten Zirkondioxid mittels Schleifen, Eckradien von mindestens 0.25 mm auf.

Bedingt durch die Anatomie des menschlichen Gebisses, hat ein Implantat im Durchschnitt einen Gesamtdurchmesser von 3 bis 6 mm, sodass mit den derzeit geschliffenen Eckradien ein Materialverlust zwischen 0.4 bis 0.6 mm bezogen auf den Durchmesser resultiert. Weitere Nachteile der Schleiftechnik bestehen bei der Gestaltung der Innenbohrung des Implantats. Derzeit besteht beispielsweise nur die Möglichkeit einen innenliegenden Dreikant im Implantat mit einem Eckradius von 0.4 mm zu schleifen, die Realisierung von kleineren Radien gestaltet sich als wirtschaftlich schwer umsetzbar.

Ein Grund für die Einschränkungen bei der Schleiftechnik ist, dass Schleifkörner an Eckradien präferentiell ausbrechen. Je schärfer, sprich je kleiner ein Eckradius ausgestaltet werden soll, desto schneller kommt es zum Ausbruch der Körner. Werkzeuge mit Radien unter 0.2 mm weisen daher einen so hohen Verschleiss auf, dass eine wirtschaftliche und masshaltige Fertigung kaum möglich ist.

Seit langem besteht daher das Bedürfnis, die Designfreiheit bei der Konstruktion von Keramikimplantaten zu erhöhen.

Es besteht insbesondere das Erfordernis, die Eckradien der Implantate und Abutments klein auszuführen um das Rotationsspiel, welches zwischen diesen beiden Teilen auftritt, zu verringern um dadurch die Positionsstabilität des Abutments im Implantat zu verbessern. Bei den bisher hergestellten Zahnersatzsystemen konnte im Durchschnitt ein Rotationsspiel von etwa 5° zwischen Abutment und Implantat festgestellt werden, welches bei Reduktion der Eckradien entsprechend minimiert werden kann.

Neben den drei vorgängig erwähnten Verfahren zur Herstellung von Zahnersatzsystemen ist zur Feinbearbeitung von Keramik die Laserabtragung bekannt. In der Dentaltechnik kommt dieses Verfahren bei der computerunterstützten Herstellung von Zahnkronen zum Einsatz.

In der EP1352619A1 wird ein solches Verfahren beschrieben, bei welchem ein Ultrakurzpulslaser vorgeschlagen wird um möglichst naturgetreu die Form des Zahnes nach zu bilden. Der offenbarte Prozess wird zweistufig ausgeführt. In einem ersten Schritt erfolgt die "grobe Bearbeitung" durch Schneiden, wobei die Oberflächengestaltung und Geometrie des Keramikrohlings an die herzustellende Oberfläche des Zahnersatzes angenähert wird. Im zweiten Schritt wird durch eine erodierende Arbeitsweise der Rest der Oberfläche vergast. Beide Prozessschritte, jener des Schneidens und jener des Erodierens werden durch unterschiedliche Betriebsweisen des Lasers ausgeführt.

In der US21014/0162210 A1 wird ein Verfahren zur Herstellung einer Innenstruktur als Eindrehgeometrie und/oder als Verdrehsicherung eines Keramikimplantates oder eines keramischen Aufbauelementes beschrieben. Dabei wird eine rotierende Scheibe zur Abtragung von Keramikmaterial in einer Vortriebrichtung der rotierenden Scheibe senkrecht zur Rotationsachse der Scheibe in eine proximal zugängliche Fläche des Keramikimplantates oder in eine Endfläche eines keramischen Aufbauelementes vorgetrieben. Die US21014/0162210 A1 betrifft ferner auch ein Keramikimplantat und ein keramischesAufbauelement, welche mit dem Verfahren hergestellt sind.

### DARSTELLUNG DER ERFINDUNG

Aufgabe der vorliegenden Erfindung ist es, ein Zahnersatzsystem aus Zirkondioxid zur Verfügung zu stellen, welches die Nachteile von bekannten Zahnersatzsystemen nicht aufweist.

Es ist eine weitere Aufgabe der Erfindung ein Verfahren zur Herstellung von Zahnersatzsystemen zur Verfügung zu stellen mit welchem möglichst kleine Eckradien, vorzugsweise kleiner als 0.15 mm, vorzugsweise kleiner 0.10 mm realisiert werden können.

Durch die vorliegende Erfindung, wie sie in den Patentansprüchen gekennzeichnet ist, wird eine neue Lösung für das vorgenannte Problem und die daraus abgeleitete Aufgaben angegeben.

Ist im Folgenden von einem Zahnersatzsystem die Rede, sind darunter folgende Teile zu verstehen: ein erstes und ein zweites zahnprothetisches Versorgungsteil, wobei es sich beim ersten zahnprothetischen Versorgungsteil um ein Teil handelt, welches im Knochen verankert wird, beispielsweise ein Implantat. Beim zweiten zahnprothetischen Versorgungsteil, handelt es sich um ein Teil, welches mit dem ersten zahnprothetischen Teil verbunden wird.

Darunter fallen: Abutments, Heilkappen, Abdruckpfosten und Gingivaformer. Des weiteren fallen darunter Schrauben und Stifte welche dazu eingerichtet sind, die Teile der definitiver oder der provisorischen Versorgung mit dem Implantat direkt zu verbinden. Das Implantat und die damit verbundenen Teile, insbesondere das Abutment sind aus hochfestem, heiss-isostatisch gepresstem mit Yttrium dotierten Zirkondioxid gefertigt.

Ist im Zusammenhang mit dem Verfahren zur Herstellung, insbesondere der kleinen Eckradien von einem Grundkörper die Rede, so ist darunter ein Rohling aus bereits gesintertem Zirkondioxid zu verstehen, welcher durch Bearbeiten mittels eines Bohrers oder Fräsers aus Volldiamant und/oder mittels Ultrakurzpulslasers die gewünschte Ausbildung der Eckradien erfährt.

Unter den Eckradien angeordnet am ersten zahnprothetischen Versorgungsteil, sind jene Radien zu verstehen welche mit Eckradien angeordnet am zweiten zahnprothetischen Versorgungsteil korrespondieren, d.h. zusammenwirken bzw. einander berühren.

Unter einem Bohrwerkzeug oder Fräswerkzeug aus Volldiamant sind Werkzeuge zu verstehen, welche aus einem polykristallinen Diamant (PKD), einem durch chemische Gasphasenabscheidung hergestellten Diamant (CVD) oder einem monokristalinem Diamant (MKD) bestehen. Unter einem mittels chemischer Gasphasenabscheidung hergestellter Diamant, ist ein Volldiamant und kein Werkzeug zu verstehen, welches lediglich durch das CVD-Verfahren mittels Diamant beschichtet wurde.

In einer weiteren zum Volldiamant alternativen Ausführungsform für das Bohr-, und Fräswerkzeug, besteht dieses aus kubischem Bornitrid (CBN).

Unter einem Ultrakurzpulslaser ist ein Laser zu verstehen, welcher ein gepulstes Laserlicht mit Pulsdauer im Bereich von Pikosekunden oder kürzer aussendet. Es handelt sich um einen Pikosekundenlaser.

Das Ausgangsmaterial in welches die Eckradien eingebracht werden, ist Zirkondioxid im bereits gesinterten Zustand. Es handelt sich nicht um einen Grünkörper der bearbeitet und erst im Anschluss einem Sinterungsprozess unterzogen wird. Beim verwendeten Material handelt es sich um Yttrium-stabilisierte TZP ("tetragonal zirconia polycrystal"), TZP-A (Yttriumoxid teilstabilisiertes Zirkonoxid) und ATZ (Alumniumoxidverstärktes Zirkonoxid) mit einer Mohs'schen Härte von ca. 8.

Der Unterschied zwischen dem bisher angewendeten Schleifen zum in der vorliegenden Erfindung umgesetzten Fräsens bzw. Bohrens ist, dass gemäss dem erfindungsgemässen Verfahren eine spanabhebende Bearbeitung des Grundkörpers aus gesintertem, hartem Zirkondioxid erfolgt. Dadurch lassen sich sehr zeitaufwendige Bearbeitungsverfahren, wie zum Beispiel ein Schleifen eines konkav ausgebildeten Eckradius in einer Aufnahmeöffnung eines Implantats, vermeiden. Die Bearbeitung der gesinterten Zirkondioxidkeramik unterliegt dann nicht mehr den Einschränkungen der Schleiftechnik sondern richtet sich nach der Metallbearbeitung. Der für das Fräsen eingesetzte Fräskopf bzw. der für das Bohren eingesetzte Bohrkopf weist einen Durchmesser von 0.5 - 5 mm auf und ist aus Volldiamant, vorzugsweise einem polykristallinen Diamant gefertigt. Der Fräskopf bzw. der Bohrkopf weist verglichen mit einem Schleifwerkzeug eine Oberflächengeometrie auf, welche eine spanabhebende Bearbeitung der Zirkondioxidkeramik ermöglicht. Die Oberflächengeometrie ist dabei so ausgestaltet, dass kleine Eckradien, unter 0.15 mm, vorzugsweise kleiner 0.10 mm an den Grundkörpern realisiert werden können.

Vorteilhaft an den sehr kleinen Eckradien ist, dass beispielsweise bei der Ausgestaltung einer Innenbohrung des Implantats grössere Wandstärken erhalten bleiben, als dies bei geschliffenen Eckradien der Fall ist, sodass die mechanische Stabilität des Implantats erhöht wird. Vergleichbares gilt für die Ausgestaltung eines Abutmentstamms.

Ein weiterer Vorteil ist die Erhöhung der Designfreiheit bei der Ausgestaltung der zahnprothetischen Versorgungsteile. Dies resultiert weiter in höherer mechanischer Festigkeit aufgrund eines geringeren Materialabtrags. Eine verbesserte Handhabung bei der Positionierung der Versorgungsteile, erhöhte chirurgische Freiheit, erhöhte Freiheit bei der prothetischen Versorgung und damit einhergehend bessere ästhetische Resultate.

Ein Vorteil des Einsatzes eines Ultrakurzpulslasers ist die berührungsfreie Bearbeitung des Grundkörpers aus Zirkondioxids. Verglichen zu einer Bearbeitung mittels Schleifen, resultiert kein Verschleiss des Bearbeitungswerkzeugs. Dies garantiert eine hohe Dimensionsstabilität und eine damit einhergehende präzise Fertigung der Teile des Zahnersatzsystems. Vergleichbares gilt für den Einsatz des Fräsers bzw. Bohrers aus Volldiamant. Der geringe Verschleiss dieser Werkzeuge während der Bearbeitung, ermöglicht eine exakte Herstellung der erwünschten Geometrie.

Ein weiterer Vorteil ist die hohe Dimensionsstabilität durch berührungsfreie Bearbeitung im Falle des Lasers (daher kein Verschleiss) und, im Falle des Fräsens, den extrem geringen Verschleiss des Volldiamants.

Das erfindungsgemässe Zahnersatzsystem umfasst mindestens ein erstes und ein zweites zahnprothetisches Versorgungsteil aus Zirkondioxid (ZrO₂). Beide Versorgungsteile weisen je Eckradien auf, wobei diese Eckradien miteinander korrespondieren, in dem das erste Versorgungsteil in einer Wirkverbindung mit dem zweiten Versorgungsteil steht. Mindestens einer der Eckradien des ersten zahnprothetischen Versorgungsteils und/oder des zweiten zahnprothetischen Versorgungsteils weisen einen Wert von kleiner als 0.15, vorzugsweise kleiner 0.1mm auf, wobei die Erstellung des mindestens einen Eckradius durch Abtragen mittels eines Laserverfahrens und/oder durch eine Bearbeitung mittels eines Bohrwerkzeugs und/oder Fräswerkzeugs aus Volldiamant oder kubischem Bornitrid erfolgt.

In einer weiteren Ausführungsform der Erfindung weist der mindestens eine Eckradius einen Wert zwischen 0.05 mm und 0.1 mm auf.

In einer Ausführungsform des erfindungsgemässen Zahnersatzsystems handelt es sich bei mindestens einem Eckradius des ersten und/oder des zweiten zahnprothetischen Versorgungsteils um einen Eckradius mit einer konkaven Geometrie.

In einer weiteren Ausführungsform weist mindestens ein Eckradius des ersten und/oder des zweiten zahnprothetischen Versorgungsteils eine um 45° zu einer horizontalen Ebene abgeschrägte Form auf.

In einer weiteren Ausführungsform der Erfindung handelt es sich beim ersten zahnprothetischen Teil um ein Implantat und die Eckradien des Implantats korrespondieren mit den Eckradien des mindestens zweiten zahnprothetischen Versorgungsteils, einem Abutment.

In einer weiteren Ausführungsform der Erfindung sind die Eckradien des Implantats durch Innenwände einer Aufnahmeöffnung des Implantats gebildet und die Eckradien des Abutments sind an einem Abutmentstamm angeordnet.

In einer weiteren Ausführungsform der Erfindung bilden die Innenwände einen Schlüsselangriff. Der Abutmentstamm weist Positionierungselemente auf, wobei die Eckradien des Schlüsselangriffs und die Eckradien der Positionierungselemente korrespondieren und der Schlüsselangriff mit den Positionierungselementen eine formschlüssige Wirkverbindung bildet, sodass das Abutment im Implantat drehstabil positionierbar ist. Als Schlüsselangriff ist jener Teil der Aufnahmeöffnung des Implantats zu verstehen, welcher mit Positionierungselementen, angeordnet am Abutmentstamm, beispielsweise ausgebildet als Vorsprünge zusammenwirkt, um auf diese Weise das Abutment zum Implantat auszurichten. Bei den Eckradien angeordnet im Schlüsselangriff und bei den Eckradien angeordnet an den Positionierungselementen spricht man von Indexierungsradien. In einer Ausführungsform ist der Schlüsselangriff an einem distalen Ende der Aufnahmeöffnung des Implantats angeordnet.

In einer weiteren Ausführungsform der Erfindung bilden die Innenwände der Aufnahmeöffnung eine Hinterschneidung und der Abutmentstamm weist Fixierungselemente auf. Die Eckradien der Hinterschneidung und die Eckradien der Fixierungselemente korrespondieren. Die Hinterschneidung bildet mit den Fixierungselementen eine formschlüssige Wirkverbindung, sodass das Abutment im Implantat in einer axialen Richtung fixierbar ist. Bei den Eckradien angeordnet an der Hinterschneidung und angeordnet an den Fixierungselementen spricht man von Einstichradien. Unter einer Hinterschneidung ist im vorliegenden Zusammenhang, ein Teil der Aufnahmeöffnung des Implantats zu verstehen, welcher proximal zum Schlüsselangriff angeordnet ist. In die Hinterschneidung greift mindestens ein Fixierungselement, angeordnet am Abutmentstamm ein. Das mindestens eine Fixierungselement ist am Abutmentstamm proximal zu den Positionierungselementen angeordnet. Ist das Abutment mit dem Implantat verbunden, greift das Fixierungselement in die Hinterschneidung ein und das Abutment ist in Richtung einer vertikalen Achse fixiert.

In einer weiteren Ausführungsform der Erfindung bilden die Innenwände der Aufnahmeöffnung mindestens eine Passzylinderöffnung und der Abutmentstamm weist einen Passzylinder auf. Die Eckradien der Passzylinderöffnung und des Passzylinders korrespondieren und Passzylinder und Passzylinderöffnung bilden eine formschlüssige Wirkverbindung, sodass zwischen den beiden Passzylindern ein Passsitz resultiert.

In einer weiteren Ausführungsform der Erfindung weist die Aufnahmeöffnung des Implantats einen in distaler Richtung gelegenen umlaufenden Rand mit einem Eckradius auf.

In einer weiteren Ausführungsform der Erfindung verläuft ein Eckradius umlaufend am Übergang zwischen dem Abutmentkopf und dem Abutmentstamm.

In einem erfindungsgemässen Verfahren zur Herstellung des erfindungsgemässen Zahnersatzsystems erfolgt die Formgebung des Grundkörpers der zahnprothetischen Versorgungsteile, insbesondere die der Eckradien mit einem Bohrer und/oder Fräser aus Volldiamant oder kubischem Bohrnitrid.

In einer weiteren Ausführungsvariante des erfindungsgemässen Verfahrens erfolgt die Formgebung der Grundkörper der zahnprothetischen Versorgungsteile, insbesondere die Ausbildung der gewünschten Eckradien durch Abtragung des Zirkondioxids mittels Ultrakurzpulslaser.

In einer weiteren Ausführungsvariante des erfindungsgemässen Verfahrens erfolgt die Erstellung der Eckradien des Schlüsselangriffs des Implantats und die der Eckradien der Positionierungselemente des Abutments, durch Abtragung des Zirkondioxids mittels Ultrakurzpulslaser.

In einer weiteren Ausführungsvariante des erfindungsgemässen Verfahrens erfolgt die Bearbeitung der Grundkörper der zahnprothetischen Versorgungsteile durch Bohren und/oder Fräsen mit einem Volldiamantwerkzeug und in einem weiteren Verfahrensschritt durch Abtragung mittels eines Ultrakurzpulslasers.

In einer Ausführungsvariante des erfindungsgemässen Verfahrens, kommt beim Fräsen ein Fräskopf mit einem Werkzeugdurchmesser von 0.5 mm bis 5 mm zum Einsatz oder ein Scheibenfräser mit einem Werkzeugdurchmesser von 5 mm bis 20 mm.

In einer erfindungsgemässen Ausführungsvariante zur Herstellung eines Zahnersatzsystems werden bei einem umsäumenden oder seitlichen Fräsen, vorzugsweise bei der Erstellung der Eckradien des zweiten zahnprothetischen Versorgungsteils, beim Heranziehen eines Fräskopfs mit einem Werkzeugdurchmesser von 0.5-1.5 mm, <= 15% des Werkzeugdurchmessers seitlich zugestellt.

In einer alternativen Ausführungsvariante des erfindungsgemässen Verfahrens wird ein Fräskopf mit einem Werkzeugdurchmesser von 1.5-5 mm herangezogen und <= 10%, vorzugsweise <= 7.5% des Werkzeugdurchmessers seitlich zugestellt.

In einer weiteren alternativen Ausführungsvariante wird ein Scheibenfräser mit einem Werkzeugdurchmesser von 5-20 mm herangezogen und <= 10%, vorzugsweise <= 5% des Werkzeugdurchmessers seitlich zugestellt.

Beim umsäumenden oder seitlichen Fräsen beträgt in einer Tiefe die Zustellung zwischen 50 und 150% des Werkzeugdurchmessers.

In einer weiteren erfindungsgemässen Ausführungsvariante des Verfahrens werden bei einem Nutenfräsen, vorzugsweise bei der Erstellung der Eckradien des ersten zahnprothetischen Versorgungsteils, ein Fräskopf mit einem Werkzeugdurchmesser von 0.5-1.5 mm herangezogen und eine Eingriffstiefe von <= 15% des Werkzeugdurchmessers zugestellt.

Als alternative Variante wird ein Fräskopfs mit einem Werkzeugdurchmesser von 1.5-5 mm herangezogen und eine Eingriffstiefe von <= 10%, vorzugsweise <= 7.5% des Werkzeugdurchmessers zugestellt.

Als weitere Alternative wird ein Scheibenfräsers mit einem Werkzeugdurchmesser von 5-20 mm herangezogen und eine Eingriffstiefe von <= 10%, vorzugsweise <= 5% des Werkzeugdurchmessers zugestellt.

Beim Nutenfräsen beträgt in einer Tiefe die Zustellung zwischen 50 und 150% des Werkzeugdurchmessers.

In einer Ausführungsvariante des erfindungsgemässen Verfahrens wird beim Fräsen eine Schnittgeschwindigkeit mehr als 100 m/min, vorzugsweise zwischen 10 -100 m/min , besonders bevorzugt zwischen 25-50 m/min gewählt. Der Vorschub beträgt zwischen 400-1200 mm/min, vorzugsweise zwischen 600-800 mm/min oder vorzugsweise 100-600 mm/min, besonders bevorzugt zwischen 200-400 mm/min. Beim Bohren wird eine Schnittgeschwindigkeit von 10-100 m/min, vorzugsweise 25-50 m/min gewählt. Der Vorschub beträgt zwischen 5-75 mm/min, vorzugsweise zwischen 10-20 mm/min. Optional erfolgt das Bohren mit Entspänen, sodass eine erhöhte Wärmeentwicklung vermieden wird.

Das erfindungsgemässe Zahnersatzsystem wird erhalten durch die vorgängig beschriebenen Ausführungsvarianten des erfindungsgemässen Verfahrens.

### KURZE ERLÄUTERUNG ZU DEN FIGUREN

Die Erfindung soll nachfolgend anhand von Ausführungsbeispielen im Zusammenhang mit der Zeichnung näher erläutert werden. Es zeigen:

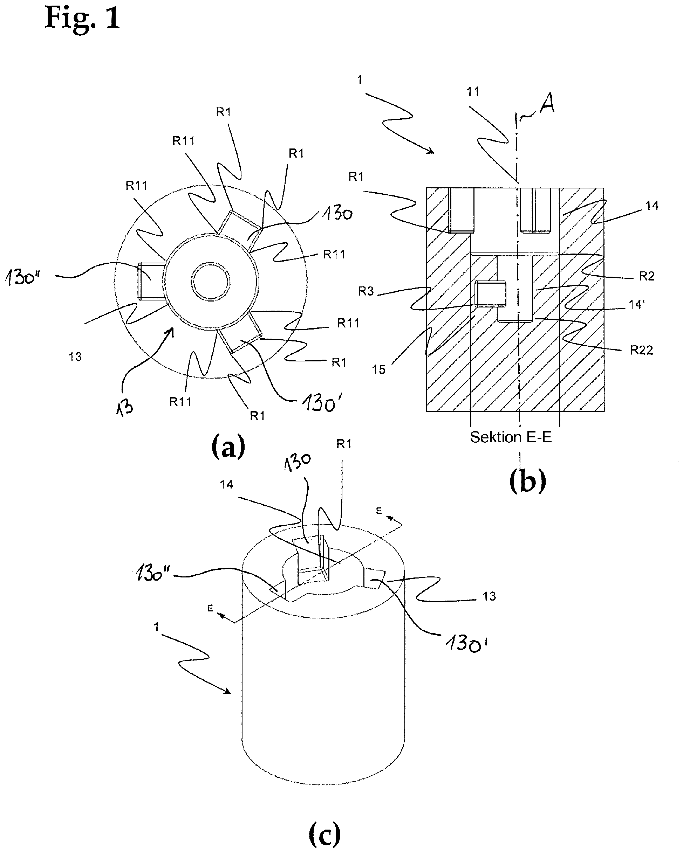

- Fig. 1: drei verschiedene schematische Ansichten, eine Ansicht von oben (a) in die Aufnahmeöffnung eine Implantats, eine Schnittdarstellung (b) der Aufnahmeöffnung, eine perspektivische Ansicht (c), wobei in allen drei Ansichten nur ein Teil des Implantats dargestellt ist, so ist beispielsweise ein Aussengewinde mit welchem das Implantat im Knochen fixiert wird nicht gezeigt,

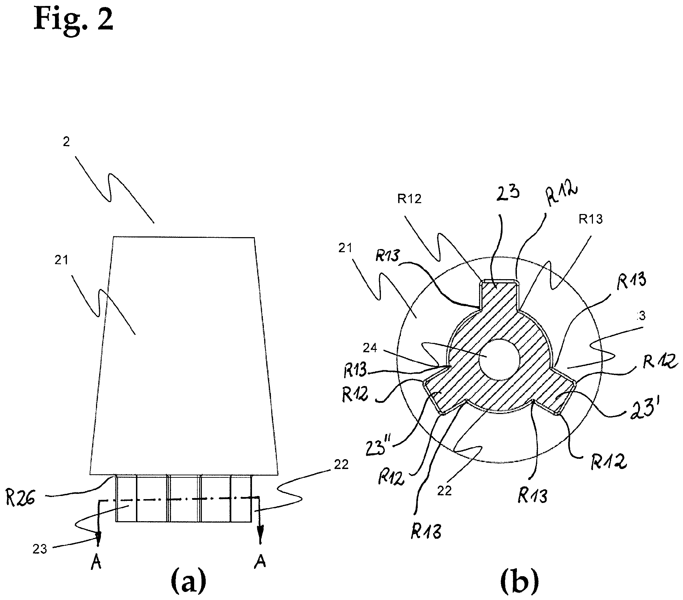

- Fig. 2: zwei verschiedene schematische Perspektiven einer gefrästen Ausführungsform eines Abutments in einer teilweisen Darstellung, unter (a) eine Seitenansicht und unter (b) eine Schnittdarstellung durch das Abutment im Bereich A-A des Abutmentstamms,

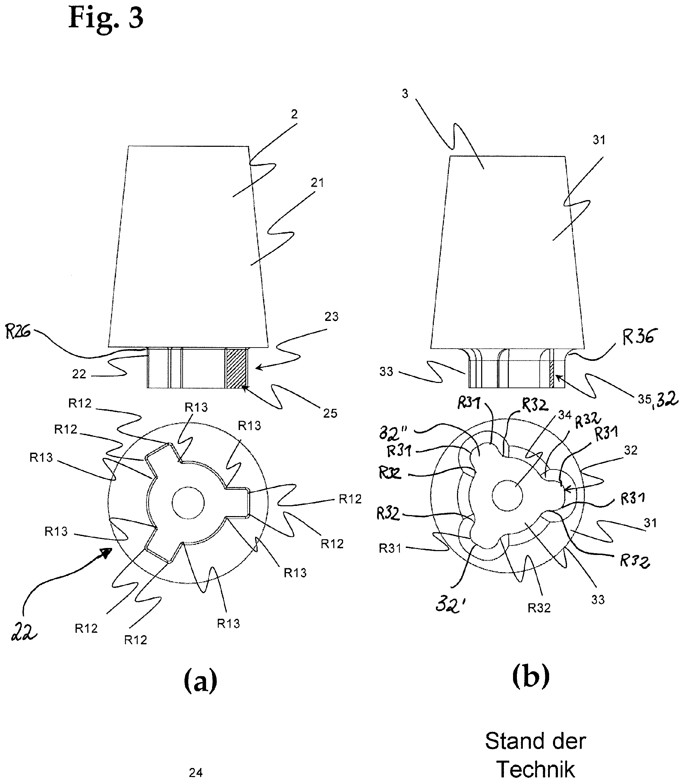

- Fig. 3: unter (a) eine weitere schematische Seitenansicht des Abutments in der gefrästen Ausführungsform gemäss Fig. 2 in einer teilweisen Darstellung sowie eine Ansicht von unten auf den Abutmentstamm und Teile des Abutmentkopfes, und unter (b) eine Seitenansicht auf ein Abutment gemäss dem Stand der Technik in einer geschliffenen Ausführungsform, sowie eine Ansicht von unten auf den Abutmentstamm und Teile eines Abutmentkopfes, wobei Sperrflächen angeordnet am Abutmentstamm schraffiert dargestellt sind,

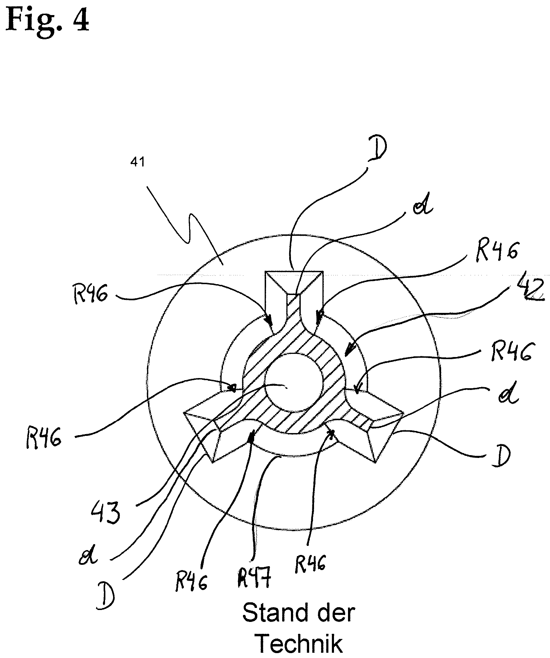

- Fig.4: ein weiteres Vergleichsbeispiel für eine aus dem Stand der Technik bekannte, geschliffene Ausführungsform eines Abutments in einer schematischen Ansicht von unten auf einen Querschnitt eines Abutmentstamm und Teile des Abutmentkopfes.

### WEGE ZUR AUSFÜHRUNG DER ERFINDUNG

Die Herstellung des erfindungsgemässen Zahnersatzsystems, insbesondere die Bearbeitung der Eckradien erfolgt mit Hilfe eines Ultrakurzpulslasers. In einer bevorzugten Ausführungsvariante des erfindungsgemässen Verfahrens beträgt die Pulsdauer des Ultrakurzpulslasers 10 ps [Pikosekunden]. Der verwendete Laser weist 10 Watt Durchschnittsleistung, eine Pulsfrequenz von 50 kHz - 8.2 MHz und eine Wellenlänge von 1064 nm [Nanometer] auf. In einer weiteren bevorzugten Ausführungsvariante beträgt die Laserleistung 8 Watt, die Pulsfrequenz 100 kHz. Eine beispielhafte Scangeschwindigkeit des Lasers liegt bei 100 mm/s.

In einer weiteren Ausführungsvariante erfolgt die Herstellung des erfindungsgemässen Zahnersatzsystems mit Hilfe eines Volldiamant-Fräsers und/oder Bohrers, welcher in eine herkömmliche CNC-Fräsmaschine eingespannt wird. In einer beispielhaften Ausführungsvariante erfolgt das Bohren mit 20 Schnittmeter (m/min) mit 2 mm/min Vorschub. Der eingesetzte Bohrer weist beispielsweise einen Durchmesser von 1.8 mm und eine Länge von 6 mm auf. In einer beispielhaften Ausführungsvariante erfolgt das Fräsen mit 20 Schnittmetern (m/min) und mit 200 mm/min Vorschub. Der eingesetzte Fräser weist beispielsweise einen Durchmesser von 1.8 mm und eine Länge von 3 mm auf.

In einer bevorzugten Ausführungsvariante sind die Bearbeitung mit Hilfe eines Ultrakurzpulslasers und die Bearbeitung mit Hilfe des Volldiamant-Fräsers/Bohrers kombiniert.

In Figur 1 (a), (b) und (c) sind drei verschiedene Perspektiven einer Ausführungsform eines Implantats 1 aus Zirkondioxid (ZrO₂) schematisch dargestellt. Die Darstellung zeigt nur einen Teil des Implantats, insbesondere eine exemplarische Ausführungsform der Innengeometrie. Ein Aussengewinde, welches in der Regel Implantate zur Befestigung im Kieferknochen aufweisen, ist in Figur 1 nicht gezeigt.

Das teilweise gezeigte Implantat 1, insbesondere dessen Innengeometrie, wurde durch Fräsen mit einem Volldiamantwerkzeug und/oder durch Laserabtragung mit Hilfe eines Ultrakurzpulslasers hergestellt. Die Aufnahmeöffnung 11 des Implantats weist Eckradien R1, R11, R2, R22 und R3 auf, welche durch die Innenwände der Aufnahmeöffnung gebildet werden. In der in Figur 1 dargestellten Ausführungsform bilden die Innenwände einen Schlüsselangriff 13, zwei passzylindrische Aufnahmeöffnungen 14, 14' und eine Hinterschneidung 15. Die Eckradien R1 sind am Schlüsselangriff 13, die Eckradien R2 und R22 an den Passzylindern und die Eckradien R3 an der Hinterschneidung angeordnet. Der Schlüsselangriff 13 dient dazu ein Abutment (in Figur 1(a) - (c) nicht gezeigt) drehstabil zu positionieren. In der in Figur 1(a), (b) und (c) dargestellten Ausführungsform ist der Schlüsselangriff 13 am distalen Ende des Implantats 1 angeordnet, wobei dieser vorzugsweise durch drei Eingriffsbereiche 130, 130' und 130" gebildet wird. Die Eckradien R1 und R11 sind an den Eingriffsbereichen 130, 130' und 130" des Schlüsselangriffs angeordnet. Die Eckradien R2, R22 und R3 liegen, in einer axialen Richtung (Achse A) gesehen, proximal zu den Eckradien R1 und R11. Bei den Eckradien R1 und R11 handelt es sich um sogenannte Indexierungsradien. Wird ein Abutment (in Fig.1 nicht gezeigt) mit dem Implantat 1 verbunden, korrespondieren diese Indexierungsradien mit weiteren Indexierungsradien angeordnet an Positionierungselementen eines Abutmentstamms. Die Eingriffsbereiche 130, 130' und 130" bilden mit den Positionierungselementen des Abutmentstamms eine formschlüssige Wirkverbindung (siehe Fig. 2, Radien R13, R12), sodass das Abutment im Implantat drehstabil positionierbar ist. Verglichen mit Implantaten und Abutments welche durch Schleifen bearbeitet wurden, resultiert nahezu kein Rotationsspiel zwischen diesen beiden Teilen. Die Eckradien R2, R22 sind an den passzylindrischen Aufnahmeöffnungen 14, 14' angeordnet und korrespondieren mit weiteren Eckradien angeordnet an einem Abutmentstamms (in Fig. 1 nicht gezeigt). Zwischen den passzylindrischen Aufnahmeöffnungen 14, 14' und den korrespondierenden Passzylindern am Abutmentstamm resultiert ein Passsitz. Bei den Eckradien R2 und R22 spricht man von sogenannten Sacklochradien. In der in Fig. 1 dargestellten Ausführungsform der Erfindung ist in proximaler Richtung zum Schlüsselangriff 13 und zur passzylindrischen Aufnahmeöffnung 14 gesehen, eine Hinterschneidung 15 angeordnet, welche Eckradien R3 aufweist. Bei den Eckradien R3 spricht man von sogenannten Einstichradien. Wird ein Abutment (in Fig. 1 nicht gezeigt) mit dem Implantat verbunden, korrespondieren die Einstichradien mit Eckradien angeordnet an Fixierungselementen des Abutmentstamms.

Die Hinterschneidung 15 und das Fixierungselement bilden eine formschlüssige Wirkverbindung, sodass das Abutment im Implantat in einer axialen Richtung fixiert ist.

In der bevorzugten Ausführungsform gemäss Figur 1(a)-(c) sind die Eckradien R1, R11, R2, R22 sowie R3 kleiner/gleich 0.15 mm, vorzugsweise kleiner/gleich 0.1 mm, besonders bevorzugt kleiner/gleich 0.05 mm.

Fig. 2 (a), (b) zeigen schematisch eine Ausführungsform eines Abutments 2 aus Zirkondioxid in einer teilweisen Darstellung, welches mit der Aufnahmeöffnung des Implantat 1, aus Fig. 1 (a)-(c) verbindbar ist. Es handelt sich um eine teilweise Darstellung des Abutments. Gezeigt ist lediglich der Kopf des Abutments 21 und Teile des Abutmentstamms 22 mit den Positionierungselementen 23, 23', 23'. Nicht gezeigt werden die am Abutmentstamm ebenfalls angeordneten Fixierungselemente und weitere Teile des Abutmentstamms, welche als Passzylinder ausgebildet sind. Die Positionierungselemente weisen Eckradien R12 und R13 auf.

Die Eckradien R1 und R11, angeordnet an den Eingriffsbereichen 130, 130', 130" des Schlüsselangriffs 13, korrespondieren mit den Eckradien R12 und R13 der Positionierungselemente. Die Eingriffsbereiche 130, 130', 130" des Schlüsselangriffs 13 und die Positionierungselemente 23, 23', 23" des Abutmentstamms bilden eine formschlüssige Wirkverbindung, sodass das Abutment 2 im Implantat 1 drehstabil positionierbar ist (in Fig. 2 nicht sichtbar).

Die Eckradien R1, R11, R12 und R13 sind kleiner/gleich 0.15 mm, vorzugsweise kleiner/gleich 0.10 mm, besonders bevorzugt kleiner 0.05 mm Des weiteren weist der Abutmentstamm 22 eine Öffnung 24, beispielsweise zur Aufnahme einer Schraube auf.

Das Abutment, insbesondere dessen Abutmentstamm 22 wurde durch ein Fräswerkzeug aus Volldiamant und/oder durch eine Laserabtragung mit Hilfe eines Ultrakurzpulslasers hergestellt.

Fig. 3(a) zeigt das Abutment 2 aus Fig. 2 in einer weiteren Seitenansicht und einer weiteren Ansicht von unten auf den Abutmentstamm 22 und den Kopf des Abutments 21. Verglichen mit der Darstellung aus der Fig. 2 ist in der Perspektive der Fig. 3(a) eine Sperrfläche 25 (schraffiert dargestellt), angeordnet an einem der drei Positionierungselemente 23, 23', 23" sichtbar.

Fig. 3(b) zeigt eine vergleichbare Perspektive auf ein Abutment wie Fig. 3(a) jedoch mit dem Unterschied, dass das Abutment durch Schleifen des Zirkondioxidkörpers auf die gewünschte Form gebracht wurde. Die daraus resultierende Geometrie des Abutments ist aus dem Stand der Technik bekannt. Dargestellt ist in der Seitenansicht das Abutment 3 mit einem Abutmentkopf 31 und einem Abutmentstamm 33. Ebenfalls sichtbar ist eine Sperrfläche 35 (schraffiert dargestellt) angeordnet an diesen Positionierungselementen 32, 32', 32". In der Darstellung des Abutments 3 in einer Sicht von unten sind die Eckradien R31 und R32 angeordnet an diesen Positionierungselementen 32, 32', 32" sichtbar. Gleich wie bei dem Abutment aus Fig.3(a) handelt es sich bei diesen Eckradien um die sogenannten Indexierungsradien.

Aus der Gegenüberstellung des erfindungsgemässen Abutments 2 und jener aus dem Stand der Technik bekannten Geometrie geht der Vorteil der kleinen Eckradien, im vorliegenden Fall jener der Indexierungsradien R12, R13, verglichen mit R31 und R32, hervor.

Die Indexierungsradien R12, R13 wurden mittels Ultrakurzpulslaser und/oder Fräsen mit Hilfe eines Volldiamantwerkzeuges hergestellt. R31 und R32 aus Fig. 3(b) sind durch Schleifen hergestellt worden.

Indexierungsradien R12, R13, R31, R32 sind sowohl am Abutmentstamm 22, 33 als auch in der Aufnahmeöffnung des Implantats vorgesehen (vgl. Fig.1, R1, R11). Der Abutmentstamm weist eine Öffnung 34 auf, welche dazu dient das Abutment 3 in einem Implantat zusätzlich zu befestigen. Wird das Abutment 2, 3 mit dem Implantat verbunden, greifen die Positionierungselemente 23, 23', 23", 32, 32', 32" in die Eingriffsbereiche 130, 130', 130" des Schlüsselangriffs ein und bilden eine formschlüssige Wirkverbindung. Bezogen auf die erfindungsgemässe Ausführungsform gemäss Fig. 3(a), korrespondieren die Eckradien R12, R13 und die Eckradien R1 und R11. Ein Rotationsspiel zwischen Abutment und dem Implantat, welches bei geschliffenen Radien im Durchschnitt 5° beträgt, kann durch die Erstellung der Eckradien angeordnet am Schlüsseleingriff und an den Positionierungselementen, mittels eines Laserverfahrens und/oder durch eine Bearbeitung mittels eines Bohrers oder Fräsers aus Volldiamant im Durchschnitt um den Faktor 5 reduziert werden.

Für die drehstabile Positionierung stehen die Sperrflächen 25 (schraffiert dargestellt) zur Verfügung, welche mit Sperrflächen angeordnet an den Eingriffsbereichen 130, 130', 130" des Schlüsselangriffs korrespondieren. Bedingt durch die sehr kleinen Eckradien von kleiner als 0.15 mm, vorzugsweise kleiner 0.1 mm verbleiben an den Positionierungselementen 23, 23', 23" entsprechend grosse Sperrflächen 25, 25', 25". Das Gleiche gilt für die Flächen der korrespondierenden Eingriffsbereiche des Schlüsselangriffs im Implantat. Die grösseren Sperrflächen, die bedingt durch die kleineren Eckradien zur Verfügung stehen, ermöglichen eine stabile Verbindung und eine verbesserte Krafteinleitung zwischen dem Abutment und dem Implantat.

Wie ein Vergleich mit der Geometrie des Abutments 3 mit jener des Abutments 2 zeigt, ist die Sperrfläche 35 wesentlich kleiner ausgebildet. Das Abutment 3 wurde mittels herkömmlichen nach dem Stand der Technik bekannten Schleifen bearbeitet.

Die Eckradien R31 und R32, welche verglichen mit den Eckradien R12 und R13 in einen Zirkondioxid-Rohkörper eingeschliffen werden, sind entsprechend grösser ausgebildet und die verbleibenden Sperrflächen 35 an den Positionierungselementen des Abutments fallen kleiner aus. Dies wirkt sich nachteilig auf die drehstabile Positionierung des Abutments im Implantat aus.

Ebenfalls in Fig. 3(a) und 3(b) sichtbar sind, zwischen dem Abutmentstamm 22, 33 und dem Abutmentkopf umlaufende Aussenradien R26 und R36. R26, welcher durch Bearbeitung mit Hilfe eines Ultrakurzpulslasers hergestellt wurde, weist einen Wert von kleiner 0.15 mm, vorzugsweise kleiner 0.1 mm auf. Ist das Abutment 2 mit einem Implantat verbunden, sitzt bedingt durch die kleinen Eckradien R26 der Kopf 2 am Implantat auf (Implantat in Fig. 3(a), (b) nicht sichtbar) und es resultiert lediglich ein minimaler umlaufender Spalt zwischen dem Abutmentkopf und einem umlaufenden, distal angeordneten Rand des Implantats.

Dies ist bei der aus dem Stand der Technik bekannten Ausführungsform eines mittels Schleifen bearbeiteten Abutments nicht der Fall. Der Radius R36 beträgt mindesten 0.2 mm. Bedingt dadurch, sitzt das Abutment im eingeführten Zustand im Implantat nicht auf dessen umlaufenden, distal angeordneten Rand auf und es resultiert ein unerwünscht grosser Spalt zwischen Abutmentkopf und Implantat.

In Fig. 4 ist eine weitere aus dem Stand der Technik bekannte, geschliffene Ausführungsform eines Abutments schematisch gezeigt.

Dargestellt ist das Abutment in einer Ansicht von unten, der Kopf des Abutments mit anschliessenden Abutmentstamm 42. Bei den Eckradien R46 handelt es sich um Aussenradien. Bei R47 handelt es sich um keinen Eckradius, sondern um jenen des Abutmentstamms 42 selbst. In dieser Ausführungsform wurde exemplarisch ein Aussenradius von 0.3 mm in den Abutmentstamm 42 eingeschliffen. Eckradien unter diesem Wert sind mittels Schleifen wirtschaftlich und technisch nur schwer umsetzbar herzustellen. Bedingt durch das Einschleifen der Eckradien reduziert sich die Materialstärke des Abutmentstamm 42 von "D" am Austritt aus dem Abutmentkopf auf "d" im gezeigten Querschnitt. Ist dieses Abutment mit dem Implantat verbunden, resultiert aufgrund der Verkleinerung der Querschnittsfläche, symbolisch dargestellt durch "D" und "d" ein Rotationsspiel zwischen dem Abutmentstamm und einer Aufnahmeöffnung im Implantat (in Fig. 4 nicht gezeigt). Dieses Rotationsspiel ist unerwünscht. Bezugszeichen 43 symbolisiert eine Durchgangsbohrung im Abutmentstamm 42, welche beispielsweise zur Aufnahme einer Verbindungsschraube dient. Bedingt durch die Reduktion der Materialstärke beim Einschleifen der Eckradien reduziert sich die Querschnittsfläche des Abutmentstamms, sodass ein Bruch des Abutmentstamms nicht auszuschliessen ist.

### Berechnungsbeispiel

Wird beispielsweise von D=0.778 mm im Bereich des Austritts des Abutmentstamms 42 vom Kopf 43 ausgegangen und wird ein Eckradius R46 von 0.3 mm eingeschliffen, so beträgt der Wert für d nur noch 0.178 mm.

Ein Vergleich der beiden aus dem Stand der Technik bekannten Geometrien, gezeigt in Fig. 3(b) und Fig. 4, verdeutlicht nochmals die Einschränkungen in der Designfreiheit von Zahnersatzsystemen aus Zirkondioxid, welche mittels Schleifen bearbeitet werden.

Ist das gemäss Figur 4 dargestellte Abutment mit einem Implantat verbunden, so würde diese wie gewünscht auf einem distal umlaufenden Rand des Implantats aufsitzen, da der Radius R46 auf das hierfür notwendige Mass geschliffen wurde. Der Querschnitt des Abutmentstamms hat sich jedoch durch den Materialabtrag soweit verringert, dass eine Stabilität des Abutments im Implantat nicht mehr gewährleistet ist.

In Fig. 3(b) ist die Positionsstabilität und die Materialstabilität im eingeführten Zustand des Abutmentstamms 33 in einem Implantats zwar sichergestellt, jedoch resultiert in diesem Zustand zwischen einem distal angeordneten Rand des Implantats und dem Abutmentkopf ein unerwünschter Spalt.

## Patentansprüche

1. Zahnersatzsystem umfassend mindestens ein erstes und ein zweites zahnprothetisches Versorgungsteil (1, 2) aus gesintertem Zirkondioxid (ZrO₂), wobei beide Versorgungsteile je Eckradien (R1, R11, R2, R22, R3, R26, R12, R13) aufweisen, diese Eckradien (R1, R11, R2, R22, R3, R26, R12, R13) miteinander korrespondieren, in dem das erste Versorgungsteil (1) in einer Wirkverbindung mit dem zweiten Versorgungsteil (2) steht, **dadurch gekennzeichnet, dass** mindestens einer der Eckradien (R1, R11, R2, R22, R3, R26, R12, R13) des ersten zahnprothetischen Versorgungsteils und/oder des zweiten zahnprothetischen Versorgungsteils ein Eckradius mit konkaver Geometrie ist und einen Wert von kleiner oder gleich 0.15 mm, vorzugsweise kleiner oder gleich 0.10 mm aufweist, wobei die Erstellung des mindestens einen Eckradius (R1, R11, R2, R22, R3, R26, R12, R13) durch Abtragen des mittels eines Laserverfahrens und/oder durch eine Bearbeitung mittels eines Bohrwerkzeugs und/oder Fräswerkzeugs aus Volldiamant oder kubischem Bornitrid erfolgt.

2. Zahnersatzsystem nach Anspruch 1, **dadurch gekennzeichnet, dass** der mindestens eine Eckradius (R1, R11, R2, R22, R3, R26, R12, R13) im Bereich zwischen 0.05 mm und 0.1 mm liegt.

3. Zahnersatzsystem nach einem der vorangegangenen Ansprüche, **dadurch gekennzeichnet, dass** es sich beim ersten zahnprothetischen Teil um ein Implantat (1) handelt und die Eckradien (R1, R11, R2, R22, R3) des Implantats mit den Eckradien (R26, R12, R13) des mindestens zweiten zahnprothetischen Versorgungsteils einem Abutment (2) korrespondieren.

4. Zahnersatzsystem nach Anspruch 3, **dadurch gekennzeichnet, dass** die Eckradien (R1, R11, R2, R22, R3) des Implantats (1) durch Innenwände einer Aufnahmeöffnung des Implantats gebildet werden und die Eckradien (R26, R12, R13) des Abutments an einem Abutmentstamm angeordnet sind.

5. Zahnersatzsystem nach Anspruch 4, **dadurch gekennzeichnet, dass** die Innenwände einen Schlüsselangriff (13) bilden und der Abutmentstamm Positionierungselemente (23, 23', 23") aufweist, wobei die Eckradien (R1, R11) des Schlüsselangriffs (13) und die Eckradien der Positionierungselemente (R12, R13) korrespondieren und der Schlüsselangriff mit den Positionierungselementen eine formschlüssige Wirkverbindung bilden, sodass das Abutment (2) im Implantat (1) drehstabil positionierbar ist.

6. Zahnersatzsystem nach einem der vorangegangenen Ansprüche 4 bis 5, **dadurch gekennzeichnet, dass** die Innenwände der Aufnahmeöffnung eine Hinterschneidung (15) bilden und der Abutmentstamm Fixierungselemente aufweist, die Eckradien der Hinterschneidung (15) des ersten zahnprothetischen Versorgungsteils und die Eckradien der Fixierungselemente des zweiten zahnprothetischen Versorgungsteils korrespondieren und die Hinterschneidung (15) mit den Fixierungselementen eine formschlüssige Wirkverbindung bilden, sodass das Abutment (2) im Implantat (1) in einer axialen Richtung fixierbar ist.

7. Zahnersatzsystem nach einem der vorangegangenen Ansprüche 3 bis 5, **dadurch gekennzeichnet, dass** die Innenwände der Aufnahmeöffnung mindestens eine Passzylinderöffnung (14, 14') bildet und der Abutmentstamm einen Passzylinder aufweist, die Eckradien der Passzylinderöffnung (14, 14') des ersten zahnprothetischen Versorgungsteils und des Passzylinders des zweiten zahnprothetischen Versorgungsteils korrespondieren und Passzylinder und Passzylinderöffnung (14, 14') eine formschlüssige Wirkverbindung bilden, sodass zwischen den beiden Passzylindern ein Passsitz resultiert.

8. Zahnersatzsystem nach einem der vorangegangen Ansprüche 4 bis 7, **dadurch gekennzeichnet, dass** die Aufnahmeöffnung des Implantats (1) einen in distaler Richtung gelegenen umlaufenden Rand mit einem Eckradius aufweist.

9. Zahnersatzsystem nach einem der vorangegangenen Ansprüche, **dadurch gekennzeichnet, dass** ein Eckradius (R26) umlaufend am Übergang zwischen dem Abutmentkopf (21) und dem Abutmentstamm (22) verläuft.

10. Zahnersatzsystem nach einem der vorangegangenen Ansprüche 1 bis 9, **dadurch gekennzeichnet, dass** es sich beim Zirkondioxid (ZrO₂) um bereits gesintertes Zirkondioxid handelt.

11. Verfahren zur Herstellung eines Zahnersatzsystems gemäss einem der vorangegangenen Ansprüche 5 bis 10 **dadurch gekennzeichnet, dass** eine Formgebung von Grundkörpern der zahnprothetischen Versorgungsteile (1, 2) und die Ausbildung gewünschter Eckradien (R1, R11, R2, R22, R3, R26, R12, R13) durch Abtragung des Zirkondioxids mittels Ultrakurzpulslaser erfolgt, wobei mindestens einer der Eckradien mit konkaver Geometrie (R1, R11, R2, R22, R3) des ersten zahnprothetischen Versorgungsteils und/oder des zweiten zahnprothetischen Versorgungsteils (R26, R1 2, R1 3) einen Wert von kleiner oder gleich 0.15 mm, vorzugsweise kleiner oder gleich 0.10 mm aufweist.

12. Verfahren zur Herstellung eines Zahnsatzsystems nach Anspruch 11, **dadurch gekennzeichnet, dass** die Erstellung der Eckradien (R1, R11) des Schlüsselangriffs (13) des Implantats (1) und die Eckradien (R12, R13) der Positionierungselemente (23, 23', 23") des Abutments (2), durch Abtragung des Zirkondioxids mittels eines Ultrakurzpulslaser, vorzugsweise eines Pikosekundenlasers erfolgt.

13. Verfahren zur Herstellung eines Zahnersatzsystems gemäss einem der vorangegangenen Ansprüche 1 bis 10, **dadurch gekennzeichnet, dass** eine Formgebung von Grundkörpern der zahnprothetischen Versorgungsteile (1, 2) und deren Eckradien (R1, R11, R2, R22, R3, R26, R12, R13) mit einem Bohrwerkzeug und/oder Fräswerkzeug aus Volldiamant oder kubischem Bornitrid erfolgt, wobei mindestens einer der Eckradien mit konkaver Geometrie (R1, R11, R2, R22, R3) des ersten zahnprothetischen Versorgungsteils und/oder des zweiten zahnprothetischen Versorgungsteils (R26, R1 2, R1 3) einen Wert von kleiner oder gleich 0.15 mm, vorzugsweise kleiner oder gleich 0.10 mm aufweist.

14. Verfahren zur Herstellung eines Zahnersatzsystems gemäss Anspruch 13, **dadurch gekennzeichnet, dass** die Bearbeitung der Grundkörper der zahnprothetischen Versorgungsteile (1, 2) in einem weiteren Verfahrensschritt durch Abtragung mittels eines Ultrakurzpulslasers erfolgt.

15. Verfahren zur Herstellung eines Zahnersatzsystems gemäss Anspruch 13 oder 14, **dadurch gekennzeichnet, dass** beim Fräsen ein Fräskopf mit einem Werkzeugdurchmesser von 0.5 mm bis 5 mm zum Einsatz kommt oder ein Scheibenfräser mit einem Werkzeugdurchmesser 5 mm bis 20 mm.

16. Verfahren zur Herstellung eines Zahnersatzsystems nach Anspruch 15, **dadurch gekennzeichnet, dass** bei einem umsäumenden oder seitlichen Fräsen, vorzugsweise bei der Erstellung der Eckradien des zweiten zahnprothetischen Versorgungsteils, beim Heranziehen eines Fräskopfs mit einem Werkzeugdurchmesser von 0.5-1.5 mm, <= 15% des Werkzeugdurchmessers seitlich zugestellt werden oder beim Heranziehen eines Fräskopfs mit einem Werkzeugdurchmesser von 1.5-5 mm, <= 10%, vorzugsweise <= 7.5% des Werkzeugdurchmessers seitlich zugestellt werden oder beim Heranziehen eines Scheibenfräsers mit einem Werkzeugdurchmesser von 5-20 mm, <= 10%, vorzugsweise <= 5% des Werkzeugdurchmessers seitlich zugestellt werden.

17. Verfahren zur Herstellung eines Zahnersatzsystems nach Anspruch 16, **dadurch gekennzeichnet, dass** in einer Tiefe die Zustellung zwischen 50 und 150% des Werkzeugdurchmessers beträgt.

18. Verfahren zur Herstellung eines Zahnersatzsystems nach einem der Ansprüche 15 bis 17, **dadurch gekennzeichnet, dass** bei einem Nutenfräsen, vorzugsweise bei der Erstellung der Eckradien des ersten zahnprothetischen Versorgungsteils, beim Heranziehen eines Fräskopfs mit einem Werkzeugdurchmesser von 0.5-1.5 mm, eine Eingriffstiefe von <= 15% des Werkzeugdurchmessers zugestellt wird oder beim Heranziehen eines Fräskopfs mit einem Werkzeugdurchmesser von 1.5-5 mm, eine Eingriffstiefe von <= 10%, vorzugsweise <= 7.5% des Werkzeugdurchmessers zugestellt wird oder beim Heranziehen eines Scheibenfräsers mit einem Werkzeugdurchmesser von 5-20 mm, eine Eingriffstiefe von <= 10%, vorzugsweise <= 5% des Werkzeugdurchmessers zugestellt wird.

19. Verfahren zur Herstellung eines Zahnersatzsystems nach Anspruch 18, **dadurch gekennzeichnet, dass** eine seitliche Zustellung bis zu 100% des Werkzeugdurchmessers beträgt.

20. Verfahren zur Herstellung eines Zahnersatzsystems nach einem der Ansprüche 13 bis 19, **dadurch gekennzeichnet, dass** beim Fräsen eine gewählte Schnittgeschwindigkeit mehr als 100 m/min, vorzugsweise zwischen 10 -100 m/min , besonders bevorzugt zwischen 25-50 m/min beträgt und ein gewählter Vorschub zwischen 400-1200 mm/min, vorzugsweise zwischen 600-800 mm/min oder vorzugsweise 100-600 mm/min beträgt, besonders bevorzugt zwischen 200-400 mm/min liegt und beim Bohren eine Schnittgeschwindigkeit von 10-100 m/min, vorzugsweise 25-50 m/min gewählt wird und der Vorschub zwischen 5-75 mm/min, vorzugsweise zwischen 10-20 mm/min liegt.

## Claims

1. Dental prosthesis system comprising at least a first and a second dental prosthetic care part (1, 2) made of sintered zirconium dioxide (ZrO₂), wherein both care parts respectively have corner radii (R1, R11, R2, R22, R3, R26, R12, R13), and these corner radii (R1, R11, R2, R22, R3, R26, R12, R13) correspond to one another when the first care part (1) is operatively connected to the second care part (2), **characterized in that** at least one of the corner radii (R1, R11, R2, R22, R3, R26, R12, R13) of the first dental prosthetic care part and/or of the second dental prosthetic care part is a corner radius of concave geometry and has a value of less than or equal to 0.15 mm, preferably less than or equal to 0.10 mm, wherein the at least one corner radius (R1, R11, R2, R22, R3, R26, R12, R13) is produced by material removal using a laser method and/or by a machining operation using a drilling tool and/or milling tool made of solid diamond or cubic boron nitride.

2. Dental prosthesis system according to Claim 1, **characterized in that** the at least one corner radius (R1, R11, R2, R22, R3, R26, R12, R13) lies in the range between 0.05 mm and 0.1 mm.

3. Dental prosthesis system according to one of the preceding claims, **characterized in that** the first dental prosthetic part is an implant (1), and the corner radii (R1, R11, R2, R22, R3) of the implant correspond to the corner radii (R26, R12, R13) of the at least second dental prosthetic care part, being an abutment (2).

4. Dental prosthesis system according to Claim 3, **characterized in that** the corner radii (R1, R11, R2, R22, R3) of the implant (1) are formed by inner walls of a receiving opening of the implant, and the corner radii (R26, R12, R13) of the abutment are arranged on an abutment stem.

5. Dental prosthesis system according to Claim 4, **characterized in that** the inner walls form a wrench socket (13), and the abutment stem has positioning elements (23, 23', 23''), wherein the corner radii (R1, R11) of the wrench socket (13) and the corner radii of the positioning elements (R12, R13) correspond, and the wrench socket and the positioning elements together form a positive operative connection, such that the abutment (2) can be positioned in the implant (1) in a rotationally stable manner.

6. Dental prosthesis system according to one of Claims 4 and 5, **characterized in that** the inner walls of the receiving opening form an undercut (15), and the abutment stem has fixing elements, the corner radii of the undercut (15) of the first dental prosthetic care part and the corner radii of the fixing elements of the second dental prosthetic care part correspond, and the undercut (15) and the fixing elements together form a positive operative connection, such that the abutment (2) can be fixed in an axial direction in the implant (1).

7. Dental prosthesis system according to one of Claims 3 to 5, **characterized in that** the inner walls of the receiving opening form at least one close-fit cylinder opening (14, 14'), and the abutment stem has a close-fit cylinder, the corner radii of the close-fit cylinder opening (14, 14') of the first dental prosthetic care part and of the close-fit cylinder of the second dental prosthetic care part correspond, and close-fit cylinder and close-fit cylinder opening (14, 14') form a positive operative connection, such that a snug fit results between the two close-fit cylinders.

8. Dental prosthesis system according to one of Claims 4 to 7, **characterized in that** the receiving opening of the implant (1) has a circumferential edge located in the distal direction and with a corner radius.

9. Dental prosthesis system according to one of the preceding claims, **characterized in that** a corner radius (R26) extends circumferentially at the transition between the abutment head (21) and the abutment stem (22).

10. Dental prosthesis system according to one of Claims 1 to 9, **characterized in that** the zirconium dioxide (ZrO₂) is already sintered zirconium dioxide.

11. Method for producing a dental prosthesis system according to one of Claims 5 to 10, **characterized in that** main bodies of the dental prosthetic care parts (1, 2) are shaped, and desired corner radii (R1, R11, R2, R22, R3, R26, R12, R13) are formed, by removing the zirconium dioxide by means of an ultrashort pulse laser, wherein at least one of the corner radii of concave geometry (R1, R11, R2, R22, R3) of the first dental prosthetic care part and/or of the second dental prosthetic care part (R26, R12, R13) has a value of less than or equal to 0.15 mm, preferably less than or equal to 0.10 mm.

12. Method for producing a dental prosthesis system according to Claim 11, **characterized in that** the corner radii (R1, R11) of the wrench socket (13) of the implant (1) and the corner radii (R12, R13) of the positioning elements (23, 23', 23'') of the abutment (2) are produced by removing the zirconium dioxide by means of an ultrashort pulse laser, preferably a picosecond laser.

13. Method for producing a dental prosthesis system according to one of Claims 1 to 10, **characterized in that** a shaping of main bodies of the dental prosthetic care parts (1, 2) and of their corner radii (R1, R11, R2, R22, R3, R26, R12, R13) is carried out using a drilling tool and/or milling tool made of solid diamond or cubic boron nitride, wherein at least one of the corner radii of concave geometry (R1, R11, R2, R22, R3) of the first dental prosthetic care part and/or of the second dental prosthetic care part (R26, R12, R13) has value of less than or equal to 0.15 mm, preferably less than or equal to 0.10 mm.

14. Method for producing a dental prosthesis system according to Claim 13, **characterized in that** the machining of the main bodies of the dental prosthetic care parts (1, 2) takes place, in a further method step, by material removal by means of an ultrashort pulse laser.

15. Method for producing a dental prosthesis system according to Claim 13 or 14, **characterized in that**, for the milling, a milling head with a tool diameter of 0.5 mm to 5 mm is used, or a side-and-face milling cutter with a tool diameter of 5 mm to 20 mm.

16. Method for producing a dental prosthesis system according to Claim 15, **characterized in that**, in a peripheral or lateral milling operation, preferably when producing the corner radii of the second dental prosthetic care part, <= 15% of the tool diameter is laterally advanced when a milling head with a tool diameter of 0.5 - 1.5 mm is used, or <= 10%, preferably <= 7.5%, of the tool diameter is laterally advanced when a milling head with a tool diameter of 1.5 - 5 mm is used, or <= 10%, preferably <= 5%, of the tool diameter is laterally advanced when a side-and-face milling cutter with a tool diameter of 5-20 mm is used.

17. Method for producing a dental prosthesis system according to Claim 16, **characterized in that** depthwise the advance amounts to between 50 and 150% of the tool diameter.

18. Method for producing a dental prosthesis system according to one of Claims 15 to 17, **characterized in that**, in a groove milling operation, preferably when producing the corner radii of the first dental prosthetic care part, a milling head with a tool diameter of 0.5 - 1.5 mm is advanced by an engagement depth of <= 15% of the tool diameter, or a milling head with a tool diameter of 1.5 - 5 mm is advanced by an engagement depth of <= 10%, preferably <= 7.5%, of the tool diameter, or a side-and-face milling cutter with a tool diameter of 5 - 20 mm is advanced by an engagement depth of <= 10%, preferably <= 5%, of the tool diameter.

19. Method for producing a dental prosthesis system according to Claim 18, **characterized in that** a lateral advance amounts to up to 100% of the tool diameter.

20. Method for producing a dental prosthesis system according to one of Claims 13 to 19, **characterized in that**, in the milling operation, a chosen cutting speed is more than 100 m/min, preferably between 10-100 m/min, particularly preferably between 25-50 m/min, and a chosen advance is between 400-1200 mm/min, preferably between 600-800 mm/min or preferably 100-600 mm/min, particularly preferably between 200-400 mm/min, and, in the drilling operation, a cutting speed of 10-100 m/min, preferably 25-50 m/min is chosen, and the advance is between 5-75 mm/min, preferably between 10-20 mm/min.

## Revendications

1. Système de prothèse dentaire comprenant au moins une première et une deuxième partie d'approvisionnement prothétique dentaire (1, 2) en dioxyde de zirconium fritté (ZrO₂), les deux parties d'approvisionnement comportant chacune des rayons d'angle (R1, R11, R2, R22, R3, R26, R12, R13), ces rayons d'angle (R1, R11, R2, R22, R3, R26, R12, R13) correspondant les uns aux autres en ce que la première partie d'approvisionnement (1) est reliée fonctionnellement à la deuxième partie d'approvisionnement (2), **caractérisé en ce qu'**au moins un des rayons d'angle (R1, R11, R2, R22, R3, R26, R12, R13) de la première partie d'approvisionnement prothétique dentaire et/ou de la deuxième partie d'approvisionnement prothétique dentaire est un rayon d'angle à géométrie concave et a une valeur inférieure ou égale à 0,15 mm, de préférence inférieure ou égale à 0,10 mm, la réalisation de l'au moins un rayon d'angle (R1, R11, R2, R22, R3, R26, R12, R13) se faisant par enlèvement au moyen d'un procédé laser et/ou par usinage au moyen d'un outil au bore et/ou d'un outil de fraisage en diamant massif ou en nitrure de bore cubique.

2. Système de prothèse dentaire selon la revendication 1, **caractérisé en ce que** l'au moins un rayon d'angle (R1, R11, R2, R22, R3, R26, R12, R13) est dans la plage comprise entre 0,05 mm et 0,1 mm.

3. Système de prothèse dentaire selon l'une des revendications précédentes, **caractérisé en ce que** la première partie prothétique dentaire est un implant (1) et les rayons d'angle (R1, R11, R2, R22, R3) de l'implant, conjointement avec les rayons d'angle (R26, R12, R13) de l'au moins une deuxième partie d'approvisionnement prothétique dentaire, correspondent à un pilier (2).

4. Système de prothèse dentaire selon la revendication 3, **caractérisé en ce que** les rayons d'angle (R1, R11, R2, R22, R3) de l'implant (1) sont formés par les parois intérieures d'une ouverture de réception de l'implant et les rayons d'angle (R26, R12, R13) du pilier sont disposés au niveau d'une tige de pilier.

5. Système de prothèse dentaire selon la revendication 4, **caractérisé en ce que** les parois intérieures forment une fixation de clé (13) et la tige de pilier comporte des éléments de positionnement (23, 23', 23''), les rayons d'angle (R1, R11) de la fixation de clé (13) et les rayons d'angle des éléments de positionnement (R12, R13) correspondant entre eux et la fixation de clé formant avec les éléments de positionnement une liaison fonctionnelle à complémentarité de formes de sorte que le pilier (2) peut être positionné dans l'implant (1) d'une manière stable en rotation.

6. Système de prothèse dentaire selon l'une des revendications précédentes 4 à 5, **caractérisé en ce que** les parois intérieures de l'ouverture de réception forment une contre-dépouille (15) et la tige de pilier comporte des éléments de fixation, les rayons d'angle de la contre-dépouille (15) de la première partie d'approvisionnement prothétique dentaire et les rayons d'angle des éléments de fixation de la deuxième partie d'approvisionnement prothétique dentaire correspondant entre eux et la contre-dépouille (15) formant avec les éléments de fixation une liaison fonctionnelle à complémentarité de formes de sorte que le pilier (2) peut être fixé dans l'implant (1) dans une direction axiale.

7. Système de prothèse dentaire selon l'une des revendications précédentes 3 à 5, **caractérisé en ce que** les parois intérieures de l'ouverture de réception forment au moins une ouverture de cylindre d'adaption (14, 14') et la tige de pilier comporte un cylindre d'adaption, les rayons d'angle de l'ouverture du cylindre d'adaption (14, 14') de la première partie d'approvisionnement prothétique dentaire et ceux du cylindre d'adaption de la deuxième partie d'approvisionnement prothétique dentaire correspondent entre eux et le cylindre d'adaption et l'ouverture de cylindre d'adaption (14, 14') forment une liaison fonctionnelle à complémentarité de formes de sorte qu'il résulte un ajustement fin entre les deux cylindres d'adaption.

8. Système de prothèse dentaire selon l'une des revendications précédentes 4 à 7, **caractérisé en ce que** l'ouverture de réception de l'implant (1) comporte un bord circonférentiel situé dans la direction distale et pourvu d'un rayon d'angle.

9. Système de prothèse dentaire selon l'une des revendications précédentes, **caractérisé en ce qu'**un rayon d'angle (R26) s'étend circonférentiellement au niveau de la transition entre la tête de pilier (21) et la tige de pilier (22).

10. Système de prothèse dentaire selon l'une des revendications précédentes 1 à 9, **caractérisé en ce que** le dioxyde de zirconium (ZrO₂) est du dioxyde de zirconium déjà fritté.

11. Procédé de fabrication d'un système de prothèse dentaire selon l'une des revendications 5 à 10 précédentes, **caractérisé en ce qu'**une mise en forme de corps de base des parties d'approvisionnement prothétique dentaire (1, 2) et la formation de rayons d'angle souhaités (R1, R11, R2, R22, R3, R26, R12 , R13) sont effectuées par enlèvement du dioxyde de zirconium à l'aide d'un laser à impulsion ultracourte, l'un au moins des rayons d'angle à géométrie concave (R1, R11, R2, R22, R3) de la première partie d'approvisionnement prothétique dentaire et/ou de la deuxième partie d'approvisionnement prothétique dentaire (R26, R12, R13) ayant une valeur inférieure ou égale à 0,15 mm, de préférence inférieure ou égale à 0,10 mm.

12. Procédé de fabrication d'un système de prothèse dentaire selon la revendication 11, **caractérisé en ce que** la réalisation des rayons d'angle (R1, R11) de la fixation de clé (13) de l'implant (1) et des rayons d'angle (R12, R13) des éléments de positionnement (23, 23', 23") du pilier (2) est effectuée par enlèvement du dioxyde de zirconium au moyen d'un laser à impulsion ultra-courte, de préférence un laser picoseconde.

13. Procédé de fabrication d'un système de prothèse dentaire selon l'une des revendications 1 à 10 précédentes, **caractérisé en ce qu'**une mise en forme de corps de base des parties d'approvisionnement prothétique dentaire (1,2) et de leurs rayons d'angle (R1, R11, R2, R22, R3, R26, R12, R13) est effectuée au moyen d'un outil de perçage et/ou de fraisage en diamant massif ou en nitrure de bore cubique, l'un au moins des rayons d'angle à géométrie concave (R1, R11, R2, R22, R3) de la première partie d'approvisionnement prothétique dentaire et/ou de la deuxième partie d'approvisionnement prothétique dentaire (R26, R12, R13) ayant une valeur inférieure ou égale à 0,15 mm, de préférence inférieure ou égale à 0,10 mm.

14. Procédé de fabrication d'un système de prothèse dentaire selon la revendication 13, **caractérisé en ce que** l'usinage des corps de base des parties d'approvisionnement prothétique dentaire (1, 2) est effectué dans une étape ultérieure du procédé par enlèvement au moyen d'un laser à impulsion ultra-courte.

15. Procédé de fabrication d'un système de prothèse dentaire selon la revendication 13 ou 14, **caractérisé en ce qu'**une tête de fraisage d'un diamètre d'outil de 0,5 mm à 5 mm ou une fraise à disque d'un diamètre d'outil de 5 mm à 20 mm est utilisée pour le fraisage.

16. Procédé de fabrication d'un système de prothèse dentaire selon la revendication 15, **caractérisé en ce que**, lors d'un fraisage en bordure ou latéral, de préférence lors de la réalisation des rayons d'angle de la deuxième partie d'approvisionnement prothétique dentaire, lors de l'utilisation d'une tête de fraisage d'un diamètre d'outil de 0,5 à 1,5 mm, inférieur ou égal à 15 % du diamètre de l'outil l'avance est effectuée latéralement ou lors de l'utilisation d'une tête de fraisage d'un diamètre d'outil de 1,5 à 5 mm, inférieur ou égal à 10 %, de préférence inférieur ou égal à 7,5 %, du diamètre de l'outil l'avance est effectuée latéralement ou lors de l'utilisation d'une fraise à disque d'un diamètre d'outil de 5 à 20 mm, inférieur ou égal à 10 % , de préférence inférieur ou égal à 5 %, du diamètre de l'outil l'avance est effectuée latéralement.

17. Procédé de fabrication d'un système de prothèse dentaire selon la revendication 16, **caractérisé en ce que**, en profondeur l'avance est comprise entre 50 et 150 % du diamètre de l'outil.

18. Procédé de fabrication d'un système de prothèse dentaire selon l'une des revendications 15 à 17, **caractérisé en ce que**, lors du fraisage d'une rainure, de préférence lors de la réalisation des rayons d'angle de la première partie d'approvisionnement prothétique dentaire, lors de l'utilisation d'une tête de fraisage d'un diamètre d'outil de 0,5 à 1,5 mm, l'avance est effectuée à une profondeur d'engagement inférieure ou égale à 15 % du diamètre de l'outil ou lors de l'utilisation d'une tête de fraisage d'un diamètre d'outil de 1,5 à 5 mm, l'avance est effectuée à une profondeur d'engagement inférieure ou égale à 10 %, de préférence inférieure ou égale à 7,5 % du diamètre de l'outil ou lors de l'utilisation d'une fraise à disque d'un diamètre d'outil de 5 à 20 mm, l'avance est effectuée à une profondeur d'engagement inférieure ou égale à 10 %, de préférence inférieure ou égale à 5 %, du diamètre de l'outil.

19. Procédé de fabrication d'un système de prothèse dentaire selon la revendication 18, **caractérisé en ce qu'**une avance latérale représente jusqu'à 100 % du diamètre de l'outil.

20. Procédé de fabrication d'un système de prothèse dentaire selon l'une des revendications 13 à 19, **caractérisé en ce que**, lors du fraisage, la vitesse de coupe choisie est de plus de 100 m/min, de préférence entre 10 à 100 m/min, de manière particulièrement préférée entre 25 à 50 m/min et l'avance choisie est comprise entre 400 et 1200 mm/min, de préférence entre 600 et 800 mm/min ou de préférence entre 100 et 600 mm/min, de manière particulièrement préférée entre 200 et 400 mm/min et, lors du perçage, la vitesse de coupe choisie est de 10 à 100 m/min, de préférence 25 à 50 m/min, et la progression est comprise entre 5 à 75 mm/min, de préférence entre 10 et 20 m/min.
